(19) Europäisches Patentamt / European Patent Office / Office européen des brevets

(11) **EP 4 279 007 A1**

(12) **EUROPEAN PATENT APPLICATION**
published in accordance with Art. 153(4) EPC

(43) Date of publication:
**22.11.2023 Bulletin 2023/47**

(21) Application number: **21919564.1**

(22) Date of filing: **19.11.2021**

(51) International Patent Classification (IPC):
**A61B 18/20** (2006.01)    **A61N 5/067** (2006.01)
**A45D 26/00** (2006.01)

(52) Cooperative Patent Classification (CPC):
**A45D 26/00; A61B 18/20; A61N 5/067**

(86) International application number:
**PCT/JP2021/042632**

(87) International publication number:
**WO 2022/153673 (21.07.2022 Gazette 2022/29)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.01.2021  JP 2021003011**

(71) Applicant: **Eidea Inc.**
**Tokyo 108-0075 (JP)**

(72) Inventor: **MURAKAMI Tomohiro**
**Tokyo 108-0075 (JP)**

(74) Representative: **Michalski Hüttermann & Partner
Patentanwälte mbB
Kaistraße 16A
40221 Düsseldorf (DE)**

(54) **DEPILATORY DEVICE AND DEPILATION METHOD**

(57)    A hair removal device performs hair removal treatment with light emitted from a light source, and includes: a light source unit including the light source; an imaging unit capable of taking an image of a treatment target area of the skin; a pore specifying unit that specifies a pore present within the treatment target area on the basis of image data of the treatment target area whose image has been taken by the imaging unit; a shift amount detecting unit that detects a shift amount of a pore position associated with a position shift of the hair removal device from the pore position of the time the image of the treatment target area has been taken; and the irradiation position correcting unit that corrects an irradiation position of light with respect to the pore on the basis of the shift amount detected by the shift amount detecting unit.

FIG. 1

EP 4 279 007 A1

**Description**

Technical Field

The present invention relates to a hair removal device and a hair removal method.

Background Art

Conventionally, there is known a laser hair removal device that irradiates body hair present on the human skin with laser light, thereby removing the body hair. All existing commercialized hair removal devices using laser light or flash lamp light apply powerful light to an entire portion of the skin to irradiate body hair whose area on the portion of the skin is at a ratio of no more than about 1% with the light; thus, they are very inefficient and are made larger, and cause more damage and risk to the skin. Furthermore, they apply the light regardless of the thickness of hair roots, the color of hair, and the color and shade of the skin, and therefore cannot be said to be ideal.

To solve these problems, in recent years, there have been proposed hair removal devices that apply laser light to only roots of body hair (such as one in Patent Literature 1). A laser hair removal device in Patent Literature 1 is configured to identify the thickness (diameter) of a hair root and the color of hair on the basis of a taken image of a portion of the skin to be treated and determine the dose of laser light to be applied on the basis of these identified hair root thickness and hair color. According to such a laser hair removal device like the one in Patent Literature 1, it is possible to apply an appropriate dose of laser light to hair to be treated, and therefore it is possible to efficiently perform hair removal.

Citation List

Patent Literature

Patent Literature 1: JP 2005-500879 A

Summary of Invention

Technical Problems

However, in the laser hair removal device described in Patent Literature 1, there is a time lag between when an image of a portion of the skin to be treated is taken and when laser light is actually applied, and in a case where the position of the laser hair removal device shifts during that time, there arises a problem that hair to be treated cannot be irradiated with the laser light.

Accordingly, an object of the present invention is to provide a hair removal device and a hair removal method that make it possible to definitely irradiate hair to be treated with a light beam.

Solution to Problems

To achieve this object, a hair removal device according to the present invention is a hair removal device that performs hair removal treatment with light emitted from a light source, and includes: a light source unit including the light source; an imaging unit capable of taking an image of a treatment target area of the skin; a pore specifying unit that specifies a pore present within the treatment target area on the basis of image data of the treatment target area whose image has been taken by the imaging unit; a shift amount detecting unit that detects a shift amount of a pore position associated with a position shift of the hair removal device from the pore position of the time the image has been taken; and an irradiation position correcting unit that corrects an irradiation position of the light with respect to the pore on the basis of the shift amount detected by the shift amount detecting unit.

The hair removal device according to the present invention may further include a movement detection unit capable of detecting a relative movement amount of the hair removal device with respect to the treatment target area, and the shift amount detecting unit may be configured to cause the movement detection unit to detect a relative movement amount of the hair removal device from the time the image has been taken with respect to the treatment target area and be able to detect the shift amount on the basis of the relative movement amount.

In the hair removal device according to the present invention, the movement detection unit may be configured to be able to detect a relative movement amount of the hair removal device in a planar direction of the treatment target area and a relative rotation amount of the hair removal device in a direction parallel to the planar direction.

In the hair removal device according to the present invention, the shift amount detecting unit may be configured to be able to detect the shift amount on the basis of first image data of an image taken for the pore specifying unit to specify a pore and second image data of an image taken again before the pore is irradiated with light.

In the hair removal device according to the present invention, the shift amount detecting unit may be configured to be able to detect the shift amount on the basis of a cutout pore image extracted from the first image data and the second image data, and the cutout pore image may be an image cut out from the first image data so as to include the pore specified by the pore specifying unit and a portion of the skin around the pore.

In the hair removal device according to the present invention, the second image data may have a smaller number of pixels than the first image data, or may have a larger pixel size than the cutout pore image.

In the hair removal device according to the present invention, the irradiation position correction unit may be configured to perform determination of whether or not to correct the irradiation position of the light in accordance with the shift amount detected by the shift amount detecting unit.

In the hair removal device according to the present invention, the irradiation position correcting unit may be configured to perform determination of whether or not to inform of an error in accordance with the shift amount detected by the shift amount detecting unit.

In the hair removal device according to the present invention, the pore specifying unit may perform specifying of the pore by AI image recognition.

Furthermore, a hair removal method according to the present invention is a hair removal method for performing hair removal treatment with light emitted from a light source, and includes: an imaging step of taking an image of a treatment target area of the skin; a pore specifying step of specifying a pore present within the treatment target area on the basis of image data of the treatment target area whose image has been taken in the imaging step; a shift amount detecting step of detecting a shift amount of a pore position from the pore position of the time the image has been taken; and an irradiation position correcting step of correcting an irradiation position of the light with respect to the pore on the basis of the shift amount detected in the shift amount detecting step.

Advantageous Effects of Invention

According to the present invention, it is possible to provide a hair removal device and a hair removal method that make it possible to definitely irradiate hair to be treated with a light beam.

Brief Description of Drawings

Fig. 1 is a diagram schematically showing a configuration of a hair removal device according to an embodiment of the present invention.
Fig. 2 is a diagram schematically showing a configuration of an irradiation position control mechanism.
Fig. 3 is a diagram schematically showing a first sensor configuration example of a movement detection sensor.
Fig. 4 is a diagram schematically showing a second sensor configuration example of the movement detection sensor.
Fig. 5 is a diagram schematically showing a third sensor configuration example of the movement detection sensor.
Fig. 6 is a diagram schematically showing a configuration of a control unit.
Fig. 7(a) is a diagram showing an original image taken by an imaging unit, and Fig. 7(b) is a diagram showing a state where pores are specified by a pore specifying unit.
Fig. 8(a) is a diagram showing image data in which a pore has been specified; Fig. 8(b) is a diagram showing a cutout pore image cut out for each specified pore; Fig. 8(c) is a diagram showing a part or all of a retaken image retaken before irradiation of a light beam; and Fig. 8(d) is a diagram showing the position of an original cutout pore image and the position of the cutout pore image in the retaken image together.
Fig. 9 is a diagram for explaining a process of detecting a device shift amount in the entire imaging area using position shift amounts of multiple position shift detection template images.
Fig. 10 is a diagram for explaining a process of detecting each shift amount with respect to each pore position using a device shift amount in the entire imaging area.
Fig. 11 is a flowchart schematically showing the flow of a hair removal method according to an embodiment of the present invention.
Fig. 12 is a flowchart schematically showing the flow from a pore specifying step to an irradiation step.
Fig. 13 is a diagram schematically showing a processing sequence of the hair removal method according to the present embodiment.
Fig. 14 is an enlarged view of a portion A in Fig. 13.

Description of Embodiment

A preferred embodiment for practicing the present invention will be described below with drawings. It is noted that the following embodiment does not limit the invention according to each claim, and all combinations of features described in the embodiment are not necessarily essential to the solution for the invention. Furthermore, the drawings are schematic diagrams drawn with exaggeration, omission, or ratio adjustment accordingly to illustrate the present invention, and thus may be different from the actual shape, positional relationship, and ratio.

A hair removal device 1 according to the present embodiment is a hair removal device that irradiates body hair present on the human skin with light from a light source, thereby removing the body hair permanently or long-term (performing hair removal treatment).

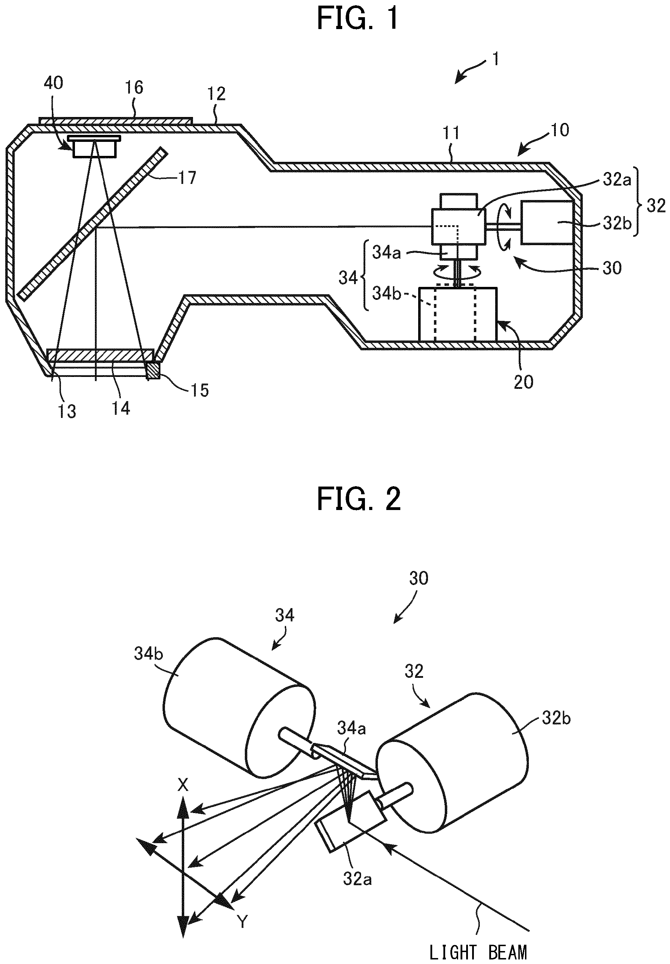

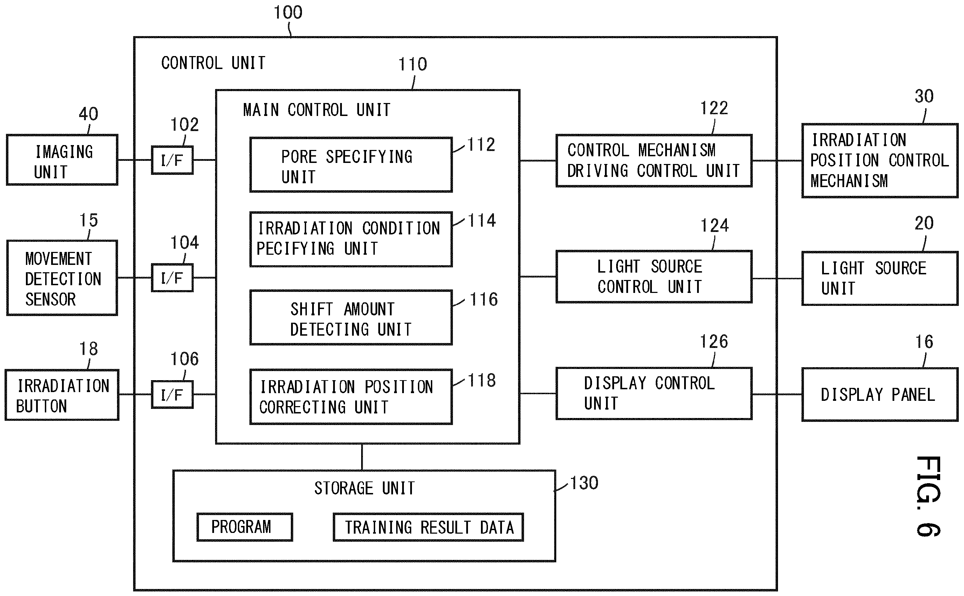

Specifically, as shown in Fig. 1, the hair removal device 1 includes: a housing 10 that a user can hold; a light source unit 20, an irradiation position control mechanism (for example, a control mechanism in which galvanometer scanners each including a rotating mirror are arranged in two directions of the X and Y axes, which makes it possible to control the light beam irradiation position in the X and Y directions) 30, and an imaging unit 40 that are housed in the housing 10; and a control unit 100 (see Fig. 6) that controls the light source unit 20 and the irradiation position control mechanism 30 on the basis of image data of an image taken by the imaging unit 40. It is noted that the control unit 100 may be provided in the housing 10, or may be provided in a separate terminal data-communicably connected to the housing 10 by wired or wireless.

[Configuration of Housing]

As shown in Fig. 1, the housing 10 includes a grip part 11 that the user can hold and a head part 12 provided continuously on the side of the distal end of the grip part 11. In the hair removal device 1 according to the present embodiment, the light source unit 20 and the irradiation position control mechanism 30 are disposed in the grip part 11, and the imaging unit 40 is disposed in the head part 12; however, their disposition is not limited to this. Furthermore, the configuration and shape of the housing 10 are not limited to an example shown in the drawing, and can be changed accordingly.

The grip part 11 is formed into an arbitrary shape such as a tubular shape having the diameter and longitudinal length that the user can hold, and is designed to have an external form fit for a skin-facing surface of the head part 12 to face a portion of the skin to be treated. Thus, with the grip part 11 held, the housing 10 makes it easy for the hair removal device 1 to be positioned on the portion of the skin to be treated, and makes it easy for the hair removal device 1 to be moved from a treated area to an untreated area. Furthermore, the grip part 11 is provided with an irradiation button 18 (see Fig. 6) for switching ON/OFF of the irradiation by the light source unit 20.

The head part 12 has an opening 13 on a skin-facing surface (in the present embodiment, a lower surface) thereof that faces a portion of the skin to be treated at the time of hair removal treatment, and is provided with a cover member 14 so as to cover the opening 13. The opening 13 has a size equal to or larger than a treatment target area of the skin to be treated by a single shot (one shot). The cover member 14 has dust resistance that can prevent entry of dust or something into the housing 10 and translucency enough not to inhibit an irradiation process performed by the light source unit 20 and an imaging process performed by the imaging unit 40. As the cover member 14, for example, a transparent glass plate or the like can be used; however, it is not limited to this.

Furthermore, a dichroic mirror 17 is provided inside the head part 12; the dichroic mirror 17 further reflects a light beam that has been emitted from the light source unit 20 and deflected by the irradiation position control mechanism 30 toward the outside of the opening 13. The dichroic mirror 17 is provided at an angle of about 45 degrees to the opening 13, and is configured to serve as a reflecting surface that efficiently reflects irradiation light that is long-wavelength infrared light so that the light beam that has been emitted from the light source unit 20 and deflected by the irradiation position control mechanism 30 is efficiently reflected toward the outside of the opening 13 (a treatment target area of the skin) by the reflecting surface. Meanwhile, unlike the irradiation light, the dichroic mirror 17 allows transmission of short-wavelength visible light therethrough with high transmittance, and the imaging unit 40 is disposed on the side of a transmission surface. Thus, the imaging unit 40 can take an image of the outside of the opening 13 (the treatment target area of the skin) through the dichroic mirror 17 with little loss.

Moreover, a lighting means (not shown) is provided inside the head part 12; the lighting means can emit illumination light toward the opening 13. The lighting means is configured to be turned on when the imaging unit 40 takes an image to illuminate a treatment target area of the skin through the opening 13. As this lighting means, various arbitrary light sources, such as a general-purpose LED, can be used.

Furthermore, on the skin-facing surface of the head part 12, a movement detection sensor 15 (a movement detection unit) is provided for detecting a relative movement amount (hereinafter, referred to as a "horizontal movement amount") of the hair removal device 1 (the head part 12) in an X-Y plane direction with respect to a portion of the skin to be treated (a treatment target area) and a relative rotation amount (hereinafter, referred to as a "horizontal rotation amount") of the hair removal device 1 (the head part 12) in a direction parallel to the X-Y plane direction. The movement detection sensor 15 is provided at a position that is not hidden by the user's hand in a state where the grip part 11 is held by the user; for example, the movement detection sensor 15 is provided near the opening 13. As the movement detection sensor 15, an optical mouse sensor, an acceleration sensor, a gyro sensor, or some other sensor can be voluntarily used. As a configuration of the movement detection sensor 15, for examples, the following first to third sensor configuration examples 15A to 15C are given.

[First Sensor Configuration Example 15A]

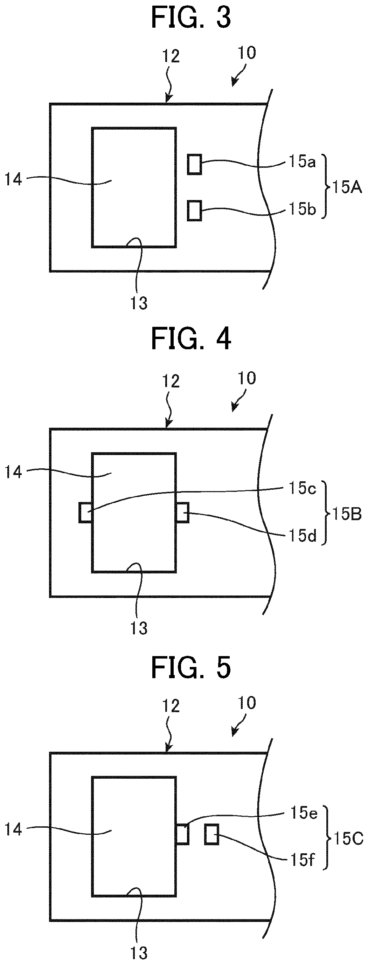

As shown in Fig. 3, the first sensor configuration example 15A is an example where a combination of an acceleration sensor 15a that can detect the acceleration of the head part 12 and a gyro sensor 15b that can detect the angular velocity of the head part 12 is used as the movement detection sensor 15. The acceleration sensor 15a and the gyro sensor 15b can be arranged in any positions near the opening 13. According to the first sensor configuration example 15A, the horizontal movement amount can be measured by integrating the acceleration detected by the acceleration sensor 15a twice, and the horizontal rotation amount can be measured by integrating the angular velocity detected by the gyro sensor 15b.

[Second Sensor Configuration Example 15B]

As shown in Fig. 4, the second sensor configuration example 15B is an example where a combination of two or more optical mouse sensors (a first optical mouse sensor 15c and a second optical mouse sensor 15d) that can detect the horizontal movement amount of the head part 12 is used as the movement detection sensor 15. The first optical mouse sensor 15c and the second optical mouse sensor 15d are preferably arranged at a distance in the X direction and/or the Y direction; in the second sensor configuration example 15B, they are arranged at a distance from each other across the opening 13. According to the second sensor configuration example 15B, the horizontal movement amount can be measured by each of the optical mouse sensors 15c and 15d, and the horizontal rotation amount can be measured by using a difference between a value measured by the first optical mouse sensor 15c and a value measured by the second optical mouse sensor 15d (a micromovement amount).

[Third Sensor Configuration Example 15C]

As shown in Fig. 5, the third sensor configuration example 15C is an example where a combination of an optical mouse sensor 15e that can detect the horizontal movement amount of the head part 12 and a gyro sensor 15f that can detect the angular velocity of the head part 12 is used as the movement detection sensor 15. The optical mouse sensor 15e and the gyro sensor 15f can be arranged in any positions near the opening 13. According to the third sensor configuration example 15C, the horizontal movement amount can be measured by the optical mouse sensor 15e, and the horizontal rotation amount can be measured by integrating the angular velocity detected by the gyro sensor 15f.

It is noted that the movement detection sensor 15 is not limited to the first to third sensor configuration examples 15A to 15C described above, and various publicly known configurations can be adopted. Furthermore, the movement detection sensor 15 may have a configuration that can detect only the horizontal movement amount.

Moreover, the head part 12 is provided with a display panel 16 on a surface (in the present embodiment, an upper surface) thereof that faces the side of the user at the time of hair removal treatment. The display panel 16 is configured to be able to display thereon a real-time image (a live image) taken by the imaging unit 40, for example, when the hair removal device 1 is moved from a treated area to an untreated area. By displaying the live image on the display panel 16 in this way, it becomes possible to help the movement of the hair removal device 1 to the untreated area. As the display panel 16, for example, a liquid crystal panel or the like can be used; however, it is not limited to this.

[Configuration of Light Source Unit]

The light source unit 20 includes a light source (not shown) of a high-brightness beam having the irradiation intensity (energy density) that can sufficiently cause damage to hair roots and remove hair permanently or long-term (perform hair removal treatment). As such a light source, for example, various publicly known light sources, such as a laser, a laser diode, a diode-excited solid-state laser, a solid-state laser, and an ultra-high-brightness LED, can be voluntarily adopted.

Preferably, a light beam emitted from the light source has a diameter required for and large enough for one hair root on an irradiated area. That is, the light beam emitted from the light source is preferably set to have a beam diameter larger than the diameter of a fair root or a pore in consideration of the image recognition accuracy, the positioning accuracy (position shift) of the scanner, etc.

The light source unit 20 is preferably configured to be able to adjust the irradiation intensity (power, dose) of the light source within a predetermined area (for example, 1 to 100 J/cm$^2$). In particular, the light source unit 20 is preferably configured to be able to select the optimal irradiation intensity according to the size of a pore of each hair to be treated, the color of the hair, and the color of the skin around the pore and emit a light beam to the hair. It is noted that as a method of controlling the irradiation intensity of the light source, various publicly known methods, such as control of the power output itself and control of the pulse width, can be adopted. Furthermore, in the present specification, the term "the size of a pore" shall include a case of indicating the size (thickness) of the pore itself, a case of indicating the diameter of a hair, and a case of indicating the total size of the pore and the hair.

Moreover, the light source unit 20 preferably includes multiple (for example, three types of) light sources having different wavelengths from one another and a multiplexing means (not shown) for appropriately combining light beams emitted from these multiple light sources together. In this case, the multiple light sources may include: a first light source (not shown) that can emit a light beam having a relatively short wavelength (for example, about 755 nm) that is likely to be absorbed by melanin pigment contained abundantly in hair; a third light source (not shown) that can emit a light beam having a relatively long wavelength (for example, about 1064 nm) that is relatively less likely to be absorbed by the melanin pigment and is gentle to the skin; and a second light source that can emit a light beam having a wavelength between those of the first and third light sources (for example, about 810 nm). Furthermore, as the multiplexing means, for example, various publicly known means, such as a wavelength-selective mirror (a dichroic mirror), a wavelength-selective prism (a dichroic prism), a polarizing beam splitter (PBS), and a polarizing plate, can be adopted.

According to such a configuration, the light sources of multiple wavelengths can emit combined beams of light at any intensity; therefore, it is possible to select not only the irradiation intensity but also a combination of optimal wavelengths according to information of the size of a pore of each hair to be treated, the color of the hair, and the color of the skin around the pore and emit light beams to the hair.

It is noted that the light source unit 20 is disposed in the grip part 11 of the housing 10 in the example shown in Fig. 1; however, its disposition is not limited to this, and the light source unit 20 can be disposed in any position in the housing 10 as long as light can be emitted from the opening 13 of the housing 10 through the irradiation position control mechanism 30 and some other components.

[Configuration of Irradiation Position Control Mechanism]

The irradiation position control mechanism 30 is a light beam deflection means (scanning means) for aiming a light beam emitted from the light source unit 20 at any position (X, Y) on a treatment target area (an X-Y plane that is an area to be subjected to treatment) of the skin. Specifically, as shown in Figs. 1 and 2, the irradiation position control mechanism 30 includes: an X-direction deflection unit 34 for moving a light beam emitted from the light source unit 20 in the X direction (a first direction) on the treatment target area of the skin; and a Y-direction deflection unit 32 for moving the light beam in the Y direction (a second direction perpendicular to the first direction) on the treatment target area of the skin.

As shown in Figs. 1 and 2, the Y-direction deflection unit 32 and the X-direction deflection unit 34 include reflecting mirrors 32a and 34a that can reflect a light beam and driving units 32b and 34b that change the tilt angles of the reflecting mirrors 32a and 34a, respectively. The Y-direction deflection unit 32 is disposed so as to reflect a light beam emitted from the light source unit 20 toward the X-direction deflection unit 34, and the X-direction deflection unit 34 is disposed so as to further reflect the light beam reflected by the Y-direction deflection unit 32 toward the dichroic mirror 17. Furthermore, the Y-direction deflection unit 32 and the X-direction deflection unit 34 are disposed so that a rotation shaft of the reflecting mirror 32a of the Y-direction deflection unit 32 and a rotation shaft of the reflecting mirror 34a of the X-direction deflection unit 34 are at right angles to each other. With this configuration, the irradiation position control mechanism 30 is configured to control the tilt angles of the reflecting mirrors 32a and 34a of the X-direction deflection unit 34 and the Y-direction deflection unit 32 and thereby can aim a light beam emitted from the light source unit 20 at any position (X, Y) on a treatment target area (an X-Y plane that is an area to be subjected to treatment) of the skin.

As the X-direction deflection unit 34 and the Y-direction deflection unit 32, for example, a galvanometer scanner (an electromagnetic method), a servomotor (an electromagnetic method), a MEMS mirror (an electromagnetic force or an electrostatic force), a deflector that tilts a mirror with an electromagnetic force or an electrostatic force, etc. can be voluntarily used, and various publicly known configurations such as an acousto-optics (AO) deflector (an acousto-optical means) can also be adopted.

[Configuration of Imaging Unit]

As shown in Fig. 1, the imaging unit 40 is disposed on the side of the transmission surface of the dichroic mirror 17, and is configured to be able to take an image of a treatment target area of the skin through the dichroic mirror 17 and the opening 13. The imaging unit 40 is preferably a 4K camera having a 4K resolution; however, it is not limited to this, and can be anything as long as it has the number of pixels that can take an image of a pore in the visual field at a sufficient resolution. As the imaging unit 40, for example, various publicly known imaging means such as a CMOS sensor, a CCD sensor, an array sensor, and a video camera tube can be voluntarily adopted.

[Configuration of Control Unit]

As shown in Fig. 6, the control unit 100 includes: external interfaces 102, 104, and 106 for connecting to devices such as the imaging unit 40, the movement detection sensor 15, and the irradiation button 18; a main control unit 110 that performs arithmetic processing for operating the hair removal device 1 and some other processing; a control mechanism driving control unit 122 that controls the irradiation position control mechanism 30; a light source control unit 124 that controls the light source unit 20; a display control unit 126 that controls the display panel 16; and a storage unit 130 that stores therein various data and information required for hair removal treatment. Furthermore, the control unit 100 further includes a communication processing unit (not shown) that can communicate with an external network.

The external interface 102 is an interface for connecting to the imaging unit 40; the external interface 104 is an interface for connecting to the movement detection sensor 15; and the external interface 106 is an interface for connecting to the irradiation button 18. It is noted that the external interfaces provided in the hair removal device 1 are not limited to these interfaces, and an external interface can be provided voluntarily in accordance with a device to connect to. Furthermore, publicly known interfaces in accordance with connected devices can be used as the external interfaces 102, 104, and 106; thus, their detailed description is omitted.

The storage unit 130 is, for example, a memory including a RAM, a ROM, etc., and stores therein a program including a command for running the main control unit 110, training result data for settings of a trained learner (a pore specifying unit 112 and an irradiation condition specifying unit 114 to be described later), etc. It is noted that the storage unit 130 may be composed of a later-described RAM and ROM included in the main control unit 110.

The main control unit 110 includes a CPU that is a hardware processor, the RAM, the ROM, etc., and is configured to expand a program stored in the storage unit 130 into the RAM, and interpret and cause the CPU to interpret and execute the program, thereby realizing functions of the pore specifying unit 112, the irradiation condition specifying unit 114, a shift amount detecting unit 116, and an irradiation position correcting unit 118 to be described later. It is noted that the CPU is preferably a high-end processor (a high-speed CPU) that can perform deep learning (DL). Furthermore, the main control unit 110 may include a plurality of hardware processors, and the hardware processors may include a GPU (including a GPU with a built-in CPU), an FPGA, or the like.

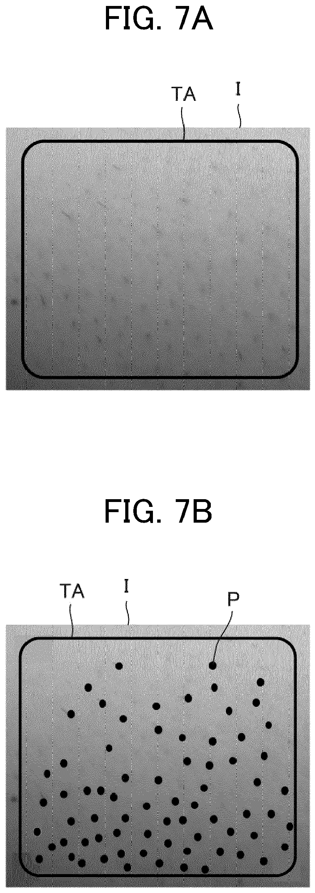

The pore specifying unit 112 is configured to specify pores present within a treatment target area on the basis of image data of the treatment target area of which the image has been taken by the imaging unit 40. Specifically, as shown in Fig. 7(a), the pore specifying unit 112 is configured to acquire image data I of a treatment target area TA of which the image has been taken by the imaging unit 40 through the external interface 102, and perform preprocessing on the image data I as necessary, and then extract candidate pores (candidate pores P) present in the treatment target area TA from the image data I by image analysis as shown in Fig. 7(b). Examples of the preprocessing include, but not be limited to, a process of performing minimum filtering on a 4K image to highlight pores, a process of decimating unnecessary information and reducing to a 2K image to lessen the burden of subsequent processes, and some other processes. It is noted that hereinafter, "image data I" ("image data" with "I" added) shall include not only the original image data of which the image has been taken by the imaging unit 40 but also the processed image data subjected to preprocessing by the pore specifying unit 112.

The extraction of candidate pores P by the pore specifying unit 112 here is preferably performed by image processing using AI, such as deep learning (DL), (AI image recognition). Specifically, the original image data is of a huge image of, for example, 4K × 2K pixels, and is not suitable for DL processing; thus, the image is divided into subregions (cells) of, for example, 256 × 256 pixels and is subjected to inference. The pore specifying unit 112 may include a trained learner (a neutral network) that has trained to minimize an objective function including an inference value of the XY coordinates of a pore in a subregion, an inference value of a degree of certainty of being a pore, etc., and may be configured to input images of subregions (cells) into which image data I of a treatment target area TA of which the image has been taken by the imaging unit 40 is divided to the learner in turn and acquire a degree of certainty and the coordinates of a candidate pore having a high degree of certainty of being a pore included in an image of a subregion from the learner, thereby extracting candidate pores P. This method does not at all include image processing by binarization that is a conventional technique, and therefore is less likely to be affected by the detection accuracy due to the brightness of a taken image, the direction of a pore, etc., and makes it possible to accurately detect pores having various different shapes and sizes. By extracting the candidate pores P by AI image recognition in this way, it becomes possible to recognize low-contrast small pores (such as those of vellus hair) that are difficult to measure, let alone detect through general image processing.

In the present embodiment, a fine-tuned convolutional neural network (for example, such as ResNet-50) trained by ImageNet or something is taken as an example of the trained learner; however, the trained learner is not limited to this. Furthermore, as the above-described objective function (an objective function including an inference value of the XY coordinates of a pore in a subregion, an inference value of a degree of certainty of being a pore, etc.), for example, the following objective function is given as an example. In the following objective function, the first term on the right side relates to the position of a pore (the XY coordinates of a pore), and is a function found by the mean square error (MSE). The second term on the right side relates to determination of whether or not there is a pore in one region, and is a function found by the binary cross entropy. The third term on the right side is a regularization term for preventing over-training.

[Formula 1]

OBJECTIVE FUNCTION :

$$loss = \lambda_{coord} \sum_{i=0}^{s^2} \sum_{j=0}^{B} \mathbb{I}_{ij}^{obj} \left[ (x_{ij} - \hat{x}_{ij})^2 + (y_{ij} - \hat{y}_{ij})^2 \right]$$

$$+ \sum_{i=0}^{s^2} \sum_{j=0}^{B} \left[ -\hat{c}_{ij} \log c_{ij} - \lambda_{noconf} (1 - \hat{c}_{ij}) \log(1 - c_{ij}) \right] + \lambda_{L2} \|w\|_2^2$$

In the above objective function (the first term on the right side), $\lambda_{coord}$ denotes a weight added to the second term on the right side in the first term on the right side, and can be fixed at, for example, 1.0 in the present embodiment. Furthermore, in the above objective function (the first term on the right side and the second term on the right side), $s^2$ denotes all cells, and B denotes all subregions in a cell.

Moreover, [Formula 2] $\mathbb{I}_{ij}^{obj}$ is an indicator function, where it returns 1 if there is an object, otherwise it returns 0; and $x_{ij}$ denotes an inference value of the X-coordinate of the position of an object in an i-cell of a j-region,

[Formula 3]

$$\hat{x}_{ij}$$

is a target of the above. Furthermore, $y_{ij}$ denotes an inference value of the Y-coordinate of the position of the object in the i-cell of the j-region,

[Formula 4]

$$\hat{y}_{ij}$$

is a target of the above.

Moreover, in the above objective function (the second term on the right side), $c_{ij}$ denotes an inference value of a degree of certainty of the presence of an object in the i-cell of the j-region,

[Formula 5]

$$\hat{c}_{ij}$$

is a target of the above. Furthermore, $\lambda_{noconf}$ denotes a weight added to a certain case in a case where there is no object, and can be set at, for example, 0.05 because in the present embodiment, there are more regions that are not pores.

Moreover, in the above objective function (the third term on the right side), $\lambda_{L2}$ denotes a weight of an L2 norm, and can be set at, for example, 0.001 in the present embodiment. Furthermore, w denotes a parameter of all kernels.

It is noted that, with respect to thick black pores and the like having sufficient contrast, instead of the configuration in which candidate pores P are extracted by AI image recognition, the pore specifying unit 112 can voluntarily adopt a method, etc. of extracting candidate pores P by a binarization process, threshold determination, etc. on image data I of a treatment target area TA of which the image has been taken by the imaging unit 40.

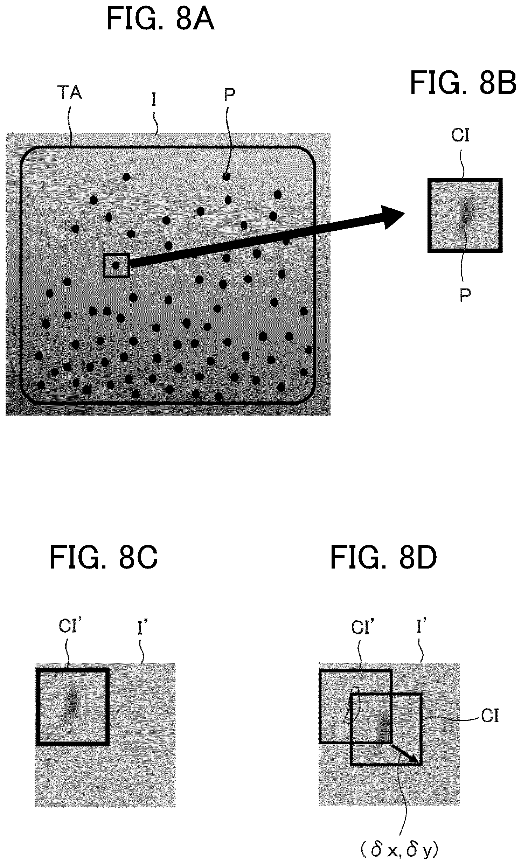

The irradiation condition specifying unit 114 is configured to specify conditions for irradiation (irradiation intensity, a wavelength, etc.) of a light beam from the light source unit 20 with respect to each pore (each candidate pore P) specified by the pore specifying unit 112. Specifically, as shown in Figs. 8(a) and 8(b), the irradiation condition specifying unit 114 is configured to, first, with respect to each pore (each candidate pore P) specified by the pore specifying unit 112, cut out an image (a cutout pore image CI) including the pore and the skin around the pore from image data I and classify the cutout pore image CI into, of multiple standard model images that differ in the size of a pore, the color of hair, the color of the skin around the pore, any one having the highest degree of certainty.

Each cutout pore image CI here is formed so that the candidate pore P is located substantially in the center thereof and the skin around the candidate pore P is present therein. Furthermore, the standard model images are an image including one or more pores and the skin around the pores, just like the cutout pore image CI. The multiple standard model images that differ in the size of a pore, the color of hair, the color of the skin around the pore are prepared in advance and stored in the storage unit 130 or some other storage. Each of the standard model images is registered and associated with optimal conditions for irradiation (irradiation intensity, a wavelength, etc.) of a light beam in terms of hair removal efficiency, safety (the risk of a burn), etc. for a target of treatment having the size of a pore, the color of hair, and the color of the skin around the pore in the standard model image. It is noted that the irradiation intensity tends to be set to a larger value as the pore diameter becomes larger, the hair color becomes lighter, and the skin color becomes paler; and the wavelength tends to be set to a shorter wavelength the pore diameter becomes larger, the hair color becomes lighter, and the skin color becomes paler.

Then, the irradiation condition specifying unit 114 is configured to specify conditions for irradiation (irradiation intensity, a wavelength, etc.) of a light beam set in advance with respect to the classified standard model image as conditions for irradiation (irradiation intensity, a wavelength, etc.) of a light beam with respect to the pore (the candidate pore P) of the cutout pore image CI.

It is noted that the classification by the irradiation condition specifying unit 114 is preferably performed by image processing using AI, such as deep learning (DL), (AI image recognition). Specifically, the irradiation condition specifying unit 114 may include a trained learner (a neutral network) that has trained to minimize an objective function including an inference value indicating the diameter of hair (the size of a pore), an inference value indicating the color of the hair, an inference value indicating the color of the skin, etc., and may be configured to input a cutout pore image CI to the learner and acquire information of a candidate pore P included in the cutout pore image CI and a standard model image having the highest degree of certainty (the highest score) from the learner, thereby classifying the cutout pore image CI into, of the multiple standard model images, the comprehensively most similar one. In this case, if all of the standard model images fall significantly below a predetermined degree of certainty, the learner may determine that none of the standard model images prepared in advance are approximate to the cutout pore image CI (i.e., none of them are classifiable), and may perform a process of determining that the candidate pore P of the cutout pore image CI is not a pore (error determination).

The shift amount detecting unit 116 is configured to detect a shift amount ($\delta$x, $\delta$y) of the pore position associated with the position shift of the hair removal device 1 from the pore position of the time an image of a treatment target area has been taken. Specifically, the shift amount detecting unit 116 is configured to detect a device shift amount ($\Delta$X, $\Delta$Y, $\Delta\theta$) of the head part 12 during a time (a time lag) from when an image of a treatment target area is taken by the imaging unit 40 until the position of a pore, conditions for irradiation with respect to the pore, etc. are specified by the pore specifying unit 112 and the irradiation condition specifying unit 114 and calculate a shift amount ($\delta$x, $\delta$y) of each pore position on the basis of that value or directly detect a shift amount ($\delta$x, $\delta$y) of each pore position. Examples of the configuration of this shift amount detecting unit 116 include a configuration in which the movement detection sensor 15 is used to detect a shift amount and a configuration in which the imaging unit 40 is used to detect a shift amount.

[Configuration in Which Movement Detection Sensor Is Used to Detect Shift Amount]

First, there is described a configuration of the shift amount detecting unit 116 in a case where the movement detection sensor 15 is used to detect a shift amount. In this case, the shift amount detecting unit 116 first detects a horizontal movement amount and a horizontal rotation amount of the head part 12 during a time from when images of all treatment target areas are taken by the imaging unit 40 until the positions of pores, conditions for irradiation with respect to the pores, etc. are specified by the pore specifying unit 112 and the irradiation condition specifying unit 114 by means of the movement detection sensor 15 (the first to third sensor configuration examples 15A to 15C, etc.). Next, a device shift amount ($\Delta X$, $\Delta Y$, $\Delta\theta$) of the hair removal device 1 in an imaging area of the full visual field of the imaging unit 40 from the device position of the time the images have been taken is calculated on the basis of the horizontal movement amount and the horizontal rotation amount, and each shift amount ($\delta x$, $\delta y$) with respect to each pore position is calculated using that value. Then, as will be described later, it is configured to make a position correction of each pore position using the shift amount identified in this way.

With this configuration, the movement of the head part 12 can be directly detected in real time by the movement detection sensor 15, and a shift amount of the head part 12 can be identified; therefore, it has the advantage that a highly real-time correction process can be realized with simple equipment.

[Configuration in Which Imaging Unit Is Used to Detect Shift Amount]

Subsequently, there is described a configuration of the shift amount detecting unit 116 in a case where the imaging unit 40 is used to detect a shift amount. Examples of the configuration of the shift amount detecting unit 116 in a case where the imaging unit 40 is used to detect a shift amount include the following first to third imaging detection configuration examples.

[First Imaging Detection Configuration Example]

The shift amount detecting unit 116 according to the first imaging detection configuration example is schematically an example where a shift amount ($\delta x$, $\delta y$) of each pore position associated with the position shift of the hair removal device 1 is directly detected on the basis of image data (first image data) of an image taken for the pore specifying unit 112 to specify a pore and image data (second image data) of an image taken again before the application of a light beam.

Specifically, first, the shift amount detecting unit 116 according to the first imaging detection configuration example registers, as a template image, a cutout pore image CI generated by the irradiation condition specifying unit 114 (or the pore specifying unit 112) in the storage unit 130. It is noted that as described above, the cutout pore image CI is an image including a pore specified by the pore specifying unit 112 and the skin around the pore that is cut out from the image data I (the first image data) of the image taken for the pore specifying unit 112 to specify the pore.

Furthermore, when the irradiation position control mechanism 30 has moved to the position of the pore specified by the pore specifying unit 112 (an intended irradiation position), or before or after that, the shift amount detecting unit 116 according to the first imaging detection configuration example causes the imaging unit 40 to retake an image of a predetermined area including the intended irradiation position. It is noted that the area of an image to retake (the pixel size of the second image data) in this case is not particularly limited as long as it is larger than the cutout pore image CI; however, from the viewpoint of high-speed processing, there is no need to perform image recognition as compared with the image data I (the first image data) of the image in the full visual field taken for the pore specifying unit 112 to specify the pore, and therefore the resolution can be a coarse resolution necessary and enough to detect a position shift (the smaller number of pixels in the same imaging area), and not the full visual field but a narrower area including the intended irradiation position (a pore to be irradiated with a light beam) is more preferable. For example, in a case where the size of the cutout pore image CI (the template image) is 64 $\times$ 64 pixels, the area of an image the imaging unit 40 retakes can be set to 128 $\times$ 128 pixels.

Then, as shown in Fig. 8 (c), the shift amount detecting unit 116 according to the first imaging detection configuration example detects a position of the cutout pore image CI' in the retaken image using various matching methods such as gray search on the basis of the cutout pore image CI and the retaken image I'. Furthermore, as shown in Fig. 8(d), the shift amount detecting unit 116 according to the first imaging detection configuration example is configured to calculate a difference between the position of the original cutout pore image CI and the position of the cutout pore image CI 'in the retaken image and identify this difference as a shift amount ($\delta x$, $\delta y$) of the pore position associated with the position shift of the hair removal device 1 from the pore position of the time the image has been taken.

According to this shift amount detecting unit 116 according to the first imaging detection configuration example, it is possible to directly take an image of a pore to be irradiated with a light beam and identify a shift amount; therefore, it has the advantage that local expansion and contraction, distortion, etc. of the skin can be corrected, and a highly accurate correction process can be realized.

[Second Imaging Detection Configuration Example]

The shift amount detecting unit 116 according to the second imaging detection configuration example is schematically an example where a device shift amount ($\Delta X$, $\Delta Y$, $\Delta\theta$) of the hair removal device 1 in an imaging area of the full visual field of the imaging unit 40 from the device position of the time an image has been taken is calculated on the basis of image data (first image data) of the image taken for the pore specifying unit 112 to specify a pore and two or more pieces of image data (second image data) of images taken again before the application of a light beam, and each shift amount ($\delta x$, $\delta y$) with respect to each pore position is detected using that value.

Specifically, first, the shift amount detecting unit 116 according to the second imaging detection configuration example registers, as position shift detection template images T1 and T2, areas of images of two or more points each including a pore image and separate from one another that have been generated by the irradiation condition specifying unit 114 (or the pore specifying unit 112) in the storage unit 130. The position shift detection template images T1 and T2 of the two or more points only have to be images at different positions from each other within a treatment target area; however, from the viewpoint of accurately detecting a horizontal movement amount and a horizontal rotation amount of the head part 12, they are preferably images at separate positions in both of the X direction and the Y direction. Furthermore, in a case where the amount of position shift is large, to prevent the pore position being falsely recognized as another pore position, the position shift detection template images T1 and T2 are desirably not of a small area including a pore at one point as in the first imaging detection configuration example but of a relatively large area including multiple pores or including a large blank space around a pore.

Next, before the irradiation of each pore with a light beam, the shift amount detecting unit 116 according to the second imaging detection configuration example causes the imaging unit 40 to retake images of predetermined areas including the position shift detection template images T1 and T2 registered in the storage unit 130. It is noted that the areas of images to retake (the pixel size of the second image data) in this case are not particularly limited as long as they are larger than the position shift detection template images T1 and T2 as in the first imaging detection configuration example.

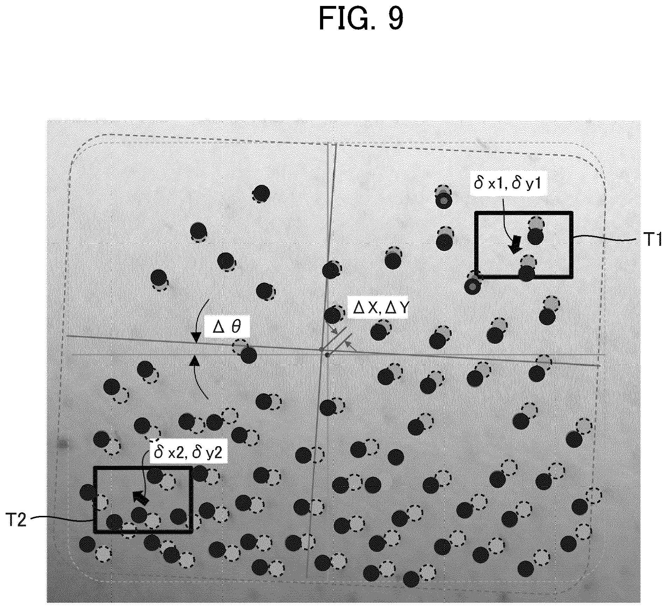

Next, as shown in Fig. 9, using various matching methods such as gray search, the shift amount detecting unit 116 according to the second imaging detection configuration example detects respective position shift amounts ($\delta x1$, $\delta y1$ and $\delta x2$, $\delta y2$) of the position shift detection template images T1 and T2.

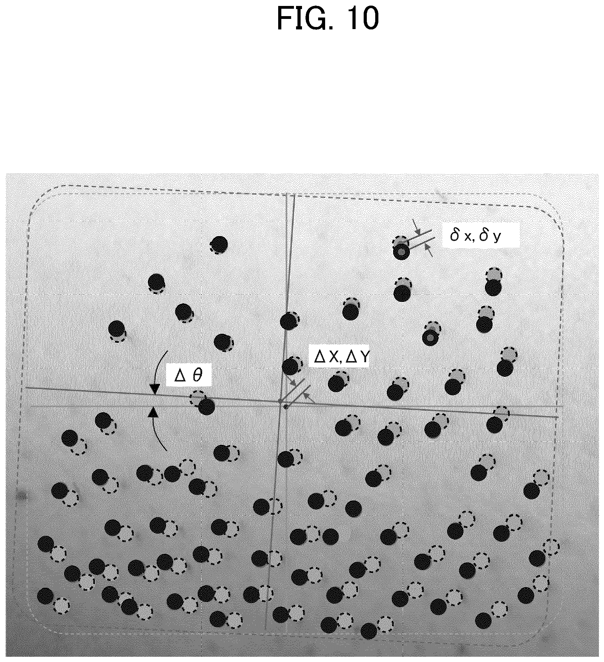

Then, the shift amount detecting unit 116 according to the second imaging detection configuration example is configured to calculate a device shift amount ($\Delta X$, $\Delta Y$, $\Delta\theta$) of the hair removal device 1 in an imaging area of the full visual field of the imaging unit 40 from the device position of the time an image has been taken using these position shift amounts ($\delta x1$, $\delta y1$ and $\delta x2$, $\delta y2$) of the two or more position shift detection template images T1 and T2 and, as shown in Fig. 10, identify each shift amount ($\delta x$, $\delta y$) of each pore position using that value.

[Third Imaging Detection Configuration Example]

The shift amount detecting unit 116 according to the third imaging detection configuration example is an example where in accordance with a similar principle of the second sensor configuration example 15B described above, two or more separate measurement areas within the visual field are extracted by means of the imaging unit 40, and, just like the above-described optical mouse sensors (the first optical mouse sensor 15c and the second optical mouse sensor 15d), the movement amounts of not only a pore but also the a micro texture of the skin are measured, and thereby the operation equivalently similar to the above-described second sensor configuration example 15B is realized.

According to this third imaging detection configuration example, just like the above-described second sensor configuration example 15B, it is possible to detect a horizontal movement amount (a position shift) and a horizontal rotation amount (a rotation shift). Furthermore, it is possible to calculate a device shift amount ($\Delta X$, $\Delta Y$, $\Delta\theta$) of the hair removal device 1 in an imaging area of the full visual field of the imaging unit 40 from the device position of the time an image has been taken on the basis of the horizontal movement amount and the horizontal rotation amount and identify each shift amount ($\delta x$, $\delta y$) of each pore position using that value. It is noted that, in the third imaging detection configuration example, to improve the contrast of the texture of the skin, a lighting with more enhanced side illumination and a lighting having a different wavelength that are different from normal illumination conditions may be used.

In a case where a shift amount is detected using the imaging unit 40 just like the shift amount detecting unit 116 according to the first to third imaging detection configuration examples described above, one sensing means (a camera) makes it possible to detect any shift of each pore within the visual field as a shift amount resulting in $\delta x$ and $\delta y$, and it is only necessary to reflect the shift amount ($\delta x$, $\delta y$) of each pore position in a control signal to the irradiation position control mechanism 30; therefore, it is not necessary to mount the sensors shown in Figs. 3 to 5, and the number of parts can be reduced.

In particular, in a case where a CMOS sensor is used in the imaging unit 40, limiting an imaging area enables high-speed imaging at a higher frame rate (for example, 1000 or more images per second), and, by using this, it becomes possible to reduce the number of parts and realize the real-time performance (responsiveness) comparable to that of the movement detection sensor 15.

It is noted that the shift amount detecting unit 116 may be configured to detect a shift amount with respect to each pore, or may be configured to detect a shift amount at intervals of a predetermined period (for every several hairs) and perform position correction of pores thinned down by reference to the latest shift amount of a nearby pore obtained not that long. That is, the frequency of detecting a shift amount by the shift amount detecting unit 116 can be set as desired.

Furthermore, in a simplified way, instead of detecting a position shift during the application of a light beam on all such occasions, it is also possible to retake an image and calculate respective shifts of one or several pores within the visual field after the irradiation of all pores within the visual field has been finished or every time the preset number of irradiations has been finished, and, only when there are more shifts than prescribed, again perform the recognition of pores and the laser irradiation, or prompt an operator to perform the operation again after an error display is made as described later.

The irradiation position correcting unit 118 is configured to identify the position to which a light beam is actually applied on the basis of the position of a pore when an image is taken (an intended irradiation position) and its shift amount later. Specifically, the irradiation position correcting unit 118 is configured to correct the position (X, Y) of a pore (i.e., intended irradiation position) specified by the pore specifying unit 112 using a shift amount ($\delta x$, $\delta y$) of each pore detected by the shift amount detecting unit 116 and identify a corrected coordinate position (X', Y') of each pore.

Furthermore, the irradiation position correcting unit 118 may be configured to determine whether or not to correct the intended irradiation position and/or determine whether or not to inform of an error according to the shift amount ($\delta x$, $\delta y$) detected by the shift amount detecting unit 116.

Specifically, the irradiation position correcting unit 118 may be configured to determine whether or not the shift amount ($\delta x$, $\delta y$) detected by the shift amount detecting unit 116 is smaller than the beam diameter and, in a case where it has been determined that the shift amount ($\delta x$, $\delta y$) is smaller than the beam diameter (i.e., in a case where the pore is irradiated with a light beam even if the light beam is applied to the intended irradiation position), not to correct the intended irradiation position. It is noted that "(being) smaller than the beam diameter" in this case includes, for example, a case where a synthetic vector of the shift amount ($\delta x$, $\delta y$) is smaller than the radius of the beam diameter; however, the criterion of the determination is not limited to this.

Furthermore, the irradiation position correcting unit 118 may be configured to determine whether or not the shift amount ($\delta x$, $\delta y$) detected by the shift amount detecting unit 116 exceeds a predetermined correctable range and, in a case where it has been determined that the shift amount ($\delta x$, $\delta y$) exceeds the correctable range, perform a warning process of informing of an error with an alarm sound or display or something and, for example, promoting re-irradiation without correcting the intended irradiation position.

Then, the irradiation position correcting unit 118 may be configured to, in a case where it has been determined that the shift amount is larger than the beam diameter (i.e., in a case where the pore is not irradiated or not sufficiently irradiated with a light beam if the light beam is applied to the intended irradiation position) and also in a case where it has been determined that the shift amount ($\delta x$, $\delta y$) is within the predetermined correctable range, correct the intended irradiation position (X, Y) on the basis of the shift amount ($\delta x$, $\delta y$).

The control mechanism driving control unit 122 is configured to control the irradiation position control mechanism 30 so that light beams from the light source unit 20 are sequentially emitted to pores one by one that have been specified by the pore specifying unit 112 of the main control unit 110 and of which the positions have been corrected by the irradiation position correcting unit 118 as necessary.

Specifically, the control mechanism driving control unit 122 is configured to sequentially control the tilt angles of the reflecting mirror 32a of the Y-direction deflection unit 32 and the reflecting mirror 34a of the X-direction deflection unit 34 so that an intended irradiation position (X, Y) of each pore specified by the pore specifying unit 112 is sequentially irradiated with a light beam in a case where the intended irradiation position has not been corrected by the irradiation position correcting unit 118. Meanwhile, the control mechanism driving control unit 122 is configured to sequentially control the tilt angles of the reflecting mirror 32a of the Y-direction deflection unit 32 and the reflecting mirror 34a of the X-direction deflection unit 34 so that a corrected coordinate position (X', Y') of each pore identified by the irradiation position correcting unit 118 is sequentially irradiated with a light beam in a case where the intended irradiation position has been corrected by the irradiation position correcting unit 118.

Such control of the control mechanism driving control unit 122 can be realized by causing, for example, a dedicated inexpensive embedded microcomputer to perform digital PID control; however, it is not limited to this.

As described above, the hair removal device 1 according to the present embodiment causes the pore specifying unit 112 to specify the position (X, Y) of each pore with high accuracy by AI image recognition, and corrects the irradiation position on the basis of a shift amount ($\delta x$, $\delta y$) of the pore position associated with a position shift of the head part 12 caused during a time from when an image is taken by the imaging unit 40 until a light beam is actually applied, and then controls the irradiation position control mechanism 30 so that the light beam is applied to each pore with pinpoint precision; therefore, it is possible to apply the light beam to only the immediate vicinity of the pore and thus improve the efficiency and the safety.

Specifically, as the high irradiation position accuracy can be secured by the above-described position correction, there is no need to increase the beam diameter more than necessary in consideration of a position shift; therefore, in a case of securing the same irradiation power density, a low-power laser can be used as a light source, and it is possible to make the device smaller and reduce costs. Furthermore, in a case of using the same light source, the irradiation time for each pore can be significantly shortened as compared with a device of a thick beam spot; therefore, it is possible to realize a high-speed hair removal device. For example, if the beam diameter can be reduced from 1 mm to 0.5 mm, the required power of the light source can be suppressed to about 1/4, and the same power as the light source can significantly shorten the irradiation time to about 1/4, and therefore can contribute to speed-up.

The light source control unit 124 is configured to control the light source unit 20 so that with respect to each pore, a light beam having irradiation conditions specified by the irradiation condition specifying unit 114 of the main control unit 110 is emitted from the light source unit 20. Specifically, the light source control unit 124 is configured to perform, with respect to each pore, control of selecting a light source (first to third light sources) to be caused to emit a light beam and output control of the light source to be caused to emit a light beam so that the light beam has the specified irradiation conditions (irradiation intensity, a wavelength, etc.). It is noted that the light source control unit 124 can also perform control of the lighting means (not shown) that can emit illumination light toward the opening 13.

The display control unit 126 is configured to be able to perform a process of transferring and displaying a real-time image (a live image) taken by the imaging unit 40 onto the display panel 16. As such a display control unit 126, various publicly known control methods can be adopted; thus, its detailed description is omitted.

[Hair Removal Method]

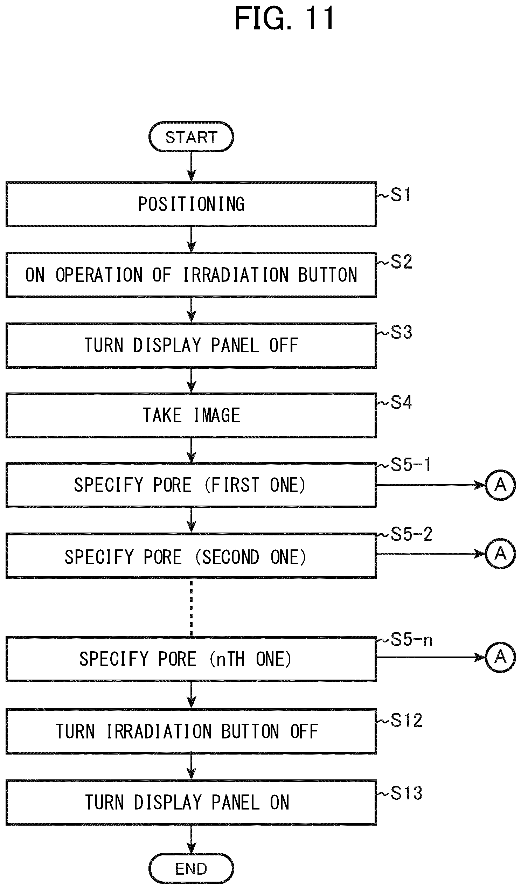

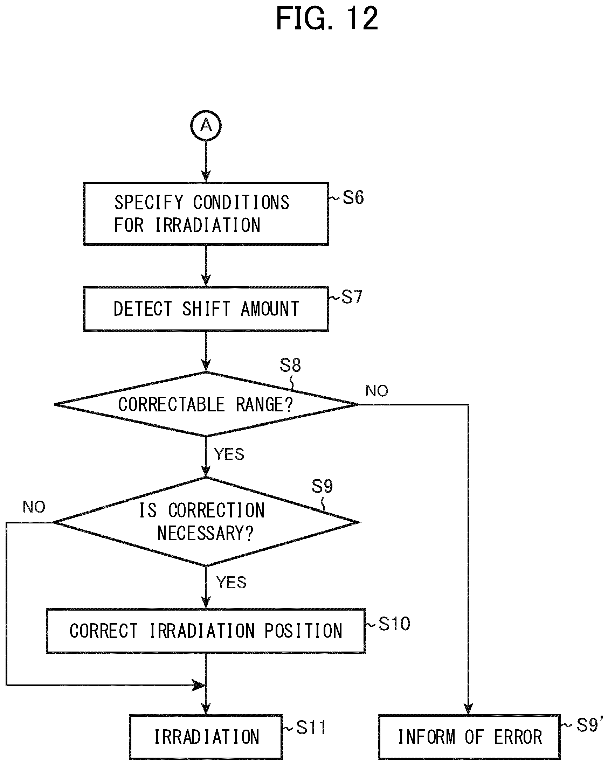

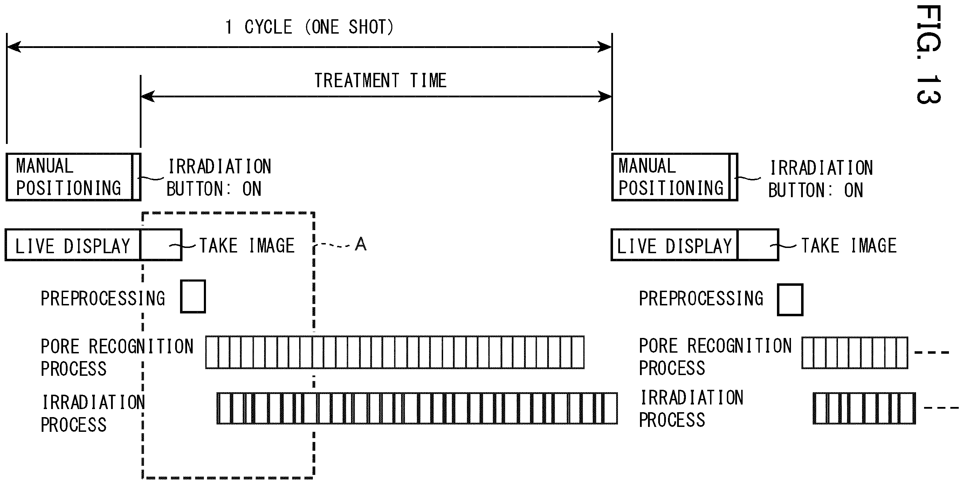

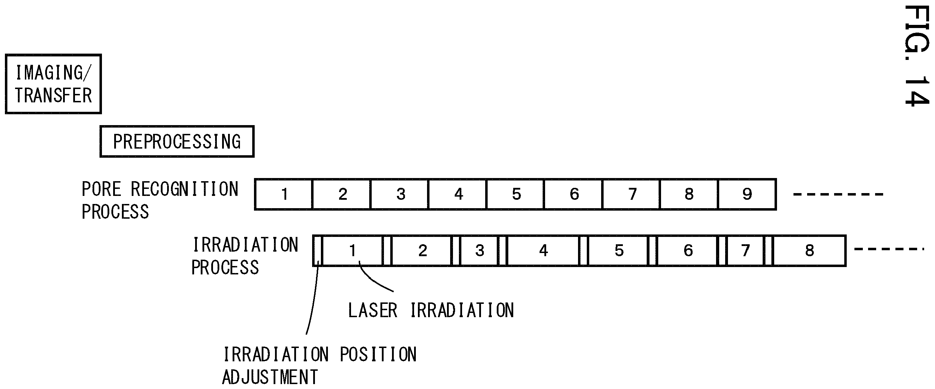

Subsequently, a hair removal method using the hair removal device 1 according to the present embodiment is described with Figs. 11 to 14. Fig. 11 is a flowchart schematically showing the overall flow of the hair removal method according to the present embodiment; Fig. 12 is a flowchart schematically showing the flow of treatment (from a pore specifying step to an irradiation step) performed on one pore specified by the pore specifying unit 112. Furthermore, Fig. 13 is a diagram schematically showing the overall processing sequence of the hair removal method according to the present embodiment; Fig. 14 is an enlarged view of a portion A shown in Fig. 13. It is noted that the hair removal method described below is implemented in accordance with the program, the training result data, etc. stored in the storage unit 130 of the hair removal device 1.

The hair removal method according to the present embodiment is schematically a hair removal method for performing hair removal treatment with light emitted from a light source, and includes: an imaging step (S4) of taking an image of a treatment target area of the skin; a pore specifying step (S5-1 to S5-n) of specifying a pore present in the treatment target area on the basis of image data of the treatment target area of which the image has been taken in the imaging step; a shift amount detecting step (S7) of detecting a shift amount ($\delta x$, $\delta y$) of the pore position from the pore position of the time the image has been taken; and an irradiation position correcting step (S10) of correcting an irradiation position (X, Y) of light with respect to the pore on the basis of the shift amount ($\delta x$, $\delta y$) detected in the shift amount detecting step.

Details of the hair removal method including these steps are described below.

When the hair removal method according to the present embodiment is started, first, the main power of the hair removal device 1 is turned ON to start the hair removal device 1. Once the hair removal device 1 is started, a real-time image (a live image) taken by the imaging unit 40 is displayed on the display panel 16. Thus, even in a state where the opening 13 is pressed against the skin (while a person is moving a shot), a treatment target area can be visually recognized through the live image on the display panel 16. It is noted that the hair removal device 1 may be manipulated by a treated person him/herself, or may be manipulated by a different person from the treated person (such as a medical worker). Hereinafter, a person who manipulates the hair removal device 1 is referred to as a "user".

In a state where the hair removal device 1 is activated, the user positions the hair removal device 1 so that the opening 13 of the housing 10 is located on a treatment target area as shown in Figs. 11 and 13 (S1), and, after completion of the positioning, performs an ON operation of the irradiation button 18 (S2). After the ON operation of the irradiation button 18 is performed, the display panel 16 is turned OFF (S3), and an image of the treatment target area of the skin is taken by the imaging unit 40 (S4: the imaging step). Then, image data of the image taken by the imaging unit 40 is transmitted to the main control unit 110 of the control unit 100, and preprocessing is performed on the image data as necessary by the function of the above-described pore specifying unit 112 in the main control unit 110, and then pores (candidate pores P) present in the treatment target area are sequentially specified (S-5 to S5-n: the pore specifying step).

Furthermore, in parallel with specifying of pores, specifying of conditions for irradiation (irradiation intensity, a wavelength, etc.), a process of correcting the irradiation position of a light beam, and an irradiation process are sequentially performed with respect to the specified pores. That is, after the first candidate pore P is specified by the function of the above-described pore specifying unit 112, as shown in Figs. 12 and 14, independently of (in parallel with) a process of specifying the second candidate pore P, the main control unit 110 performs a process of specifying conditions for irradiation and some other processes with respect to the first candidate pore P. Furthermore, after the second candidate pore P is specified, independently of (in parallel with) the process of specifying conditions for irradiation and some other processes with respect to the first candidate pore P and a process of specifying the third candidate pore P, the main control unit 110 performs a process of specifying the irradiation condition and some other processes with respect to the second candidate pore P. The main control unit 110 performs these parallel processes until the last (the nth) candidate pore P. The sequence of the recognition of a pore and the irradiation of a light beam performed in parallel in this way allows the time for the recognition process to be secured without prolonging the time taken for one cycle.

Specifying of conditions for irradiation (irradiation intensity, a wavelength, etc.) with respect to each candidate pore P (S6: an irradiation condition specifying step) is performed by the function of the above-described irradiation condition specifying unit 114 in the main control unit 110. It is noted that in the irradiation condition specifying step, in a case where it has been determined that there is no corresponding standard model image, it is determined that the candidate pore P is not a pore, and the process with respect to the candidate pore P may be terminated without moving on to the next step (without performing the irradiation of a light beam with respect to the candidate pore P).

Furthermore, after or in parallel with this irradiation condition specifying step, a shift amount ($\delta x$, $\delta y$) of the pore position from the pore position of the time the image has been taken is detected by the function of the above-described shift amount detecting unit 116 in the main control unit 110 (S7: the shift amount detecting step). It is noted that the shift amount detecting step may be performed with respect to each candidate pore P, or may be performed at intervals of a predetermined period (for every several hairs).

After the shift amount ($\delta x$, $\delta y$) is detected in the shift amount detecting step, determination of whether or not the shift amount exceeds a predetermined correctable range is performed (S8). Then, in a case where it has been determined that the shift amount exceeds the predetermined correctable range, a warning process of informing of an error with an alarm sound or display or something and, for example, promoting re-irradiation is performed (S9').

On the other hand, in a case where it has been determined that the shift amount ($\delta x$, $\delta y$) detected in the shift amount detection step is within the predetermined correctable range, determination of whether or not the shift amount needs to be corrected is performed (S9). In this determination, for example, whether or not the shift amount is smaller than the beam diameter (i.e., whether or not the pore is irradiated with a light beam even if the light beam is applied to the intended irradiation position) may be determined.

Then, in a case where it has been determined in this determination step that the correction is necessary, the irradiation position (X, Y) of light with respect to the pore is corrected on the basis of the shift amount ($\delta x$, $\delta y$) detected in the shift amount detecting step by the function of the above-described irradiation position correcting unit 118 in the main control unit 110 (S10: the irradiation position correcting step). Furthermore, after the irradiation position correcting step, the process moves on to the irradiation step (S11). On the other hand, in a case where it has been determined in the above-described determination step that the correction is not necessary, the process moves on to the irradiation step (S11) without going through the irradiation position correcting step (S10).

In the irradiation step (S11), the irradiation position control mechanism 30 is driven by the control mechanism driving control unit 122 so that a coordinate position (X, Y) of the candidate pore P specified in the pore specifying step or a corrected coordinate position (X', Y') of the candidate pore P corrected in the irradiation position correcting step is irradiated with a light beam from the light source unit 20, and the irradiation position is controlled. Then, after the irradiation position control, a light beam having the conditions for irradiation (irradiation intensity, a wavelength, etc.) specified in the irradiation condition specifying step is emitted from the light source unit 20 to the candidate pore P (S11: the irradiation step). Thus, a hair root of the candidate pore P is heated and removed permanently or long-term.

It is noted that the time required for this control of the irradiation position differs depending on conditions such as the moving distance; however, it takes roughly about a few milliseconds. Furthermore, the irradiation time of a light beam differs depending on conditions such as the irradiation intensity; however, it takes roughly about a few milliseconds to a few tens of milliseconds. The conditions for irradiation (irradiation intensity, a wavelength, etc.) of a light beam at this time are the optimal conditions for irradiation (irradiation intensity, a wavelength, etc.) assigned to the most approximate standard model image, and therefore are effective for the candidate pore P as well, and are less harmful to its surrounding skin, and are safe for the skin.

Then, a series of the processes from the pore specifying step to the irradiation step described above is performed with respect to the last (nth) candidate pore P, and when the processes with respect to all the candidate pores P are finished, as shown in Figs. 11 and 13, the irradiation button 18 is put into an OFF state (S12), and a real-time image (a live image) taken by the imaging unit 40 is again displayed on the display panel 16 (S13).

With the processes from the positioning (movement) of the hair removal device 1 until completion of the irradiation with respect to all the pores in the treatment target area described above as 1 cycle, these processes are performed on all of desired treatment target areas by sequentially shifting the position, thereby hair removal treatment is performed. It is noted that the estimated time from the ON operation of the irradiation button 18 until the completion of irradiation with respect to pores in a treatment target area (the treatment time of the hair removal device 1) in 1 cycle is 1 second or less if it is assumed that the number of pores is 30 or less and the irradiation and movement time is 20 ms, and is 3 seconds or less if it is assumed that the number of pores is 100 or less and the irradiation and movement time is 20 ms. In this way, the hair removal device 1 according to the present embodiment can perform the hair removal treatment in an extremely short time.

[Advantages of Hair Removal Device According to Present Embodiment]

As described above, the hair removal device 1 according to the present embodiment includes: the light source unit 20 including the light sources; the imaging unit 40 that can take an image of a treatment target area of the skin; the pore specifying unit 112 that specifies pores present in the treatment target area on the basis of image data of the treatment target area of which the image has been taken by the imaging unit 40; the shift amount detecting unit 116 that detects a shift amount ($\delta$x, $\delta$y) of the pore position associated with the position shift of the hair removal device 1 from the pore position of the time the image has been taken; and the irradiation position correcting unit 118 that corrects the irradiation position (X, Y) of light with respect to the pore on the basis of the shift amount ($\delta$x, $\delta$y) of each pore detected by the shift amount detecting unit 116.

The hair removal device 1 according to the present embodiment configured as described above can correct the irradiation position (X, Y) on the basis of the shift amount ($\delta$x, $\delta$y) of the pore position associated with the position shift of the hair removal device 1 caused in the time between the imaging by the imaging unit 40 and the actual irradiation of a light beam; therefore, even in a case where the relative position of the hair removal device 1 (the head part 12) to the treatment target area (the skin) is shifted after the image has been taken by the imaging unit 40, it is possible to apply a light beam to each pore with certainty and pinpoint precision. Furthermore, this makes it possible to apply a light beam to only a pore, and therefore it is possible to improve the efficiency and the safety.

[Modification Examples]

The preferred embodiment of the present invention is described above; however, the technical scope of the present invention is not limited to the scope described in the above embodiment. In the above embodiment, various modifications or improvements can be made.

For example, in the above-described embodiment, there is described a configuration in which the irradiation condition specifying unit 114 classifies a cutout pore image CI into any of the standard model images, thereby specifying the irradiation conditions according to the size of a pore, the color of hair, the color of the surrounding skin, etc.; however, it is not limited to this, and may be configured not to change the irradiation conditions with respect to each pore.

Furthermore, in the above-described embodiment, there is described a configuration in which recognition of only pores is performed by the pore specifying unit 112 for the moment, and after that, a cutout pore image CI is cut out from image data I by the irradiation condition specifying unit 114; however, it is not limited to this, and the pore specifying unit 112 may add inference values of the size of a pore, the color of hair, the color of the surrounding skin, etc. to an objective function, and thereby classifies an image similar to a standard model image or infers the position on the basis of images of subregions (cells) into which the image data I is divided, and directly acquires the position and size of the pore, the color of hair, and the color of the surrounding skin.

Moreover, in the above-described embodiment, there is described a configuration in which the cutout pore image CI is classified by AI image recognition; however, it is not limited to this, and it is possible to voluntarily adopt a method, etc. of quantifying respective feature values of the size of a candidate pore P included in the cutout pore image CI, the color of hair, and the color of the surrounding skin, and comparing these with respective feature values of the standard models registered in a database in advance, thereby classifying the cutout pore image CI into the most approximate standard model.

Furthermore, in the above-described embodiment, there is described a configuration in which the hair removal device 1 includes the trained learner (the neutral network) for performing AI image recognition associated with specifying of a pore and classification of a pore image; however, it is not limited to this, and a trained learner may be installed in a separate device connected to the hair removal device 1 via a high-speed communication network, and the hair removal device 1 may be configured to communicate with the separate device in real time (cloud computing). Moreover, it may have a configuration in which in the hair removal device 1 or the separate device, machine learning (AI learning process) is performed using the image data I of a treatment target area TA as input data and the degree of certainty of being a pore and the coordinates as reference data on the basis of a predetermined learning program or a configuration in which images taken by a plurality of the hair removal devices 1 are uploaded via the cloud, and the number of acquired images is increased in terms of speed, thereby learning data with enhanced recognition accuracy is shared in real time.

Furthermore, in the above-described embodiment, there is described a configuration in which the dichroic mirror 17 is provided inside the head part 12, and the light source unit 20 is disposed on the side of the reflecting surface of this dichroic mirror 17, and the imaging unit 40 is disposed on the side of the transmission surface; however, it is not limited to this. For example, it may have a configuration in which the imaging unit 40 is disposed on the side of the reflecting surface of this dichroic mirror 17, and the light source unit 20 is disposed on the side of the transmission surface. Furthermore, it may have a configuration in which the dichroic mirror 17 is not provided. It is noted that examples of the configuration in which the dichroic mirror 17 is not provided include a configuration in which the imaging unit 40 is disposed at right angle to the opening 13 (a treatment target area of the skin) to cause a light beam that has been emitted from the light source unit 20 and deflected by the irradiation position control mechanism 30 to be applied to the opening 13 (the treatment target area of the skin) from an oblique direction, a configuration in which the imaging unit 40 takes an image of the opening 13 (the treatment target area of the skin) from an oblique direction, and a light beam that has been emitted from the light source unit 20 and deflected by the irradiation position control mechanism 30 is caused to be applied to the opening 13 (the treatment target area of the skin) from the oblique direction; however, it is not limited to these.

It is obvious from the description of claims that the above modification examples are included in the scope of the present invention.

Reference Signs List

| | |
|---|---|
| 1 | hair removal device |
| 10 | housing |
| 11 | grip part |
| 12 | head part |
| 13 | opening |
| 14 | cover member |
| 15 | movement detection sensor |
| 15A | first sensor configuration example |
| 15B | second sensor configuration example |
| 15C | third sensor configuration example |
| 15a | acceleration sensor |
| 15b | gyro sensor |
| 15c | first optical mouse sensor |
| 15d | second optical mouse sensor |
| 15e | optical mouse sensor |
| 15f | gyro sensor |
| 16 | display panel |
| 17 | dichroic mirror |
| 18 | irradiation button |
| 20 | light source unit |
| 30 | irradiation position control mechanism |
| 32 | Y-direction deflection unit |
| 32a | reflecting mirror |
| 32b | driving unit |
| 34 | X-direction deflection unit |
| 34a | reflecting mirror |
| 34b | driving unit |
| 40 | imaging unit |
| 100 | control unit |
| 102 | external interface |
| 104 | external interface |
| 106 | external interface |
| 110 | main control unit |
| 112 | pore specifying unit |
| 114 | irradiation condition specifying unit |
| 116 | shift amount detecting unit |
| 118 | irradiation position correcting unit |
| 122 | control mechanism driving control unit |
| 124 | light source control unit |
| 126 | display control unit |

130     storage unit

**Claims**

1.   A hair removal device that performs hair removal treatment with light emitted from a light source, the hair removal device comprising:

      a light source unit including the light source;
      an imaging unit capable of taking an image of a treatment target area of a skin;
      a pore specifying unit that specifies a pore present within the treatment target area on a basis of image data of the treatment target area whose image has been taken by the imaging unit;
      a shift amount detecting unit that detects a shift amount of a pore position associated with a position shift of the hair removal device from the pore position of time the image of the treatment target area has been taken; and
      an irradiation position correcting unit that corrects an irradiation position of the light with respect to the pore on a basis of the shift amount detected by the shift amount detecting unit.

2.   The hair removal device according to claim 1, further comprising

      a movement detection unit capable of detecting a relative movement amount of the hair removal device with respect to the treatment target area, wherein
      the shift amount detecting unit is configured to cause the movement detection unit to detect a relative movement amount of the hair removal device from the time the image has been taken with respect to the treatment target area and be able to detect the shift amount on a basis of the relative movement amount.

3.   The hair removal device according to claim 2, wherein
      the movement detection unit is configured to be able to detect a relative movement amount of the hair removal device in a planar direction of the treatment target area and a relative rotation amount of the hair removal device in a direction parallel to the planar direction.

4.   The hair removal device according to any one of claims 1 to 3, wherein
      the shift amount detecting unit is configured to be able to detect the shift amount on a basis of first image data of an image taken for the pore specifying unit to specify a pore and second image data of an image taken again before the pore is irradiated with light.

5.   The hair removal device according to claim 4, wherein

      the shift amount detecting unit is configured to be able to detect the shift amount on a basis of a cutout pore image extracted from the first image data and the second image data, and
      the cutout pore image is an image cut out from the first image data so as to include the pore specified by the pore specifying unit and a portion of the skin around the pore.

6.   The hair removal device according to claim 5, wherein
      the second image data has a smaller number of pixels than the first image data, or has a larger pixel size than the cutout pore image.

7.   The hair removal device according to any one of claims 1 to 6, wherein
      the irradiation position correcting unit is configured to perform determination of whether or not to correct the irradiation position of the light in accordance with the shift amount detected by the shift amount detecting unit.

8.   The hair removal device according to any one of claims 1 to 7, wherein
      the irradiation position correcting unit is configured to perform determination of whether or not to inform of an error in accordance with the shift amount detected by the shift amount detecting unit.

9.   The hair removal device according to any one of claims 1 to 8, wherein

the pore specifying unit performs specifying of the pore by AI image recognition.

10. A hair removal method for performing hair removal treatment with light emitted from a light source, the hair removal method comprising:

an imaging step of taking an image of a treatment target area of a skin;

a pore specifying step of specifying a pore present within the treatment target area on a basis of image data of the treatment target area whose image has been taken in the imaging step;

a shift amount detecting step of detecting a shift amount of a pore position from the pore position of time the image has been taken; and

an irradiation position correcting step of correcting an irradiation position of light with respect to the pore on a basis of the shift amount detected in the shift amount detecting step.

# FIG. 1

# FIG. 2

LIGHT BEAM

## FIG. 3

12    10

14

15a  }
15b  } 15A

13

## FIG. 4

12    10

14

15c }
15d } 15B

13

## FIG. 5

12    10

14

15e }
15f } 15C

13

FIG. 6

EP 4 279 007 A1

## FIG. 7A

## FIG. 7B

# FIG. 8A

# FIG. 8B

CI

P

# FIG. 8C

CI′     I′

# FIG. 8D

CI′     I′

CI

$(\delta x, \delta y)$

# FIG. 9

FIG. 10

# FIG. 11

```
           START
             │
             ▼
┌─────────────────────────────┐
│        POSITIONING          │~S1
└─────────────────────────────┘
             │
             ▼
┌─────────────────────────────┐
│ ON OPERATION OF IRRADIATION BUTTON │~S2
└─────────────────────────────┘
             │
             ▼
┌─────────────────────────────┐
│    TURN DISPLAY PANEL OFF    │~S3
└─────────────────────────────┘
             │
             ▼
┌─────────────────────────────┐
│         TAKE IMAGE          │~S4
└─────────────────────────────┘
             │
             ▼
┌─────────────────────────────┐
│    SPECIFY PORE (FIRST ONE) │~S5-1 ───────→ (A)
└─────────────────────────────┘
             │
             ▼
┌─────────────────────────────┐
│   SPECIFY PORE (SECOND ONE) │~S5-2 ───────→ (A)
└─────────────────────────────┘
             ┊
             ▼
┌─────────────────────────────┐
│    SPECIFY PORE (nTH ONE)   │~S5-n ───────→ (A)
└─────────────────────────────┘
             │
             ▼
┌─────────────────────────────┐
│  TURN IRRADIATION BUTTON OFF │~S12
└─────────────────────────────┘
             │
             ▼
┌─────────────────────────────┐
│    TURN DISPLAY PANEL ON    │~S13
└─────────────────────────────┘
             │
             ▼
            END
```

# FIG. 12

(A)

↓

| SPECIFY CONDITIONS FOR IRRADIATION | ~S6 |

↓

| DETECT SHIFT AMOUNT | ~S7 |

↓

S8

CORRECTABLE RANGE? ——— NO ———→

YES ↓

S9

NO ←——— IS CORRECTION NECESSARY?

YES ↓

| CORRECT IRRADIATION POSITION | ~S10 |

↓

| IRRADIATION | ~S11 |     | INFORM OF ERROR | ~S9' |

27

# FIG. 13

MANUAL
POSITIONING

IRRADIATION
BUTTON: ON

LIVE DISPLAY TAKE IMAGE

PREPROCESSING

PORE RECOGNITION
PROCESS

IRRADIATION
PROCESS

1 CYCLE (ONE SHOT)

TREATMENT TIME

A

MANUAL
POSITIONING

IRRADIATION
BUTTON: ON

LIVE DISPLAY TAKE IMAGE

PREPROCESSING

PORE RECOGNITION
PROCESS

IRRADIATION
PROCESS

## FIG. 14

IMAGING/
TRANSFER

PREPROCESSING

PORE RECOGNITION
PROCESS

| 1 | 2 | 3 | 4 | 5 | 6 | 7 | 8 | 9 | -------

IRRADIATION
PROCESS

| | 1 | | 2 | 3 | | 4 | | 5 | | 6 | | 7 | | 8 | ------

LASER IRRADIATION

IRRADIATION POSITION
ADJUSTMENT

## INTERNATIONAL SEARCH REPORT

| International application No. |
|---|
| **PCT/JP2021/042632** |

**A. CLASSIFICATION OF SUBJECT MATTER**

*A61B 18/20*(2006.01)i; *A61N 5/067*(2006.01)i; *A45D 26/00*(2006.01)i
FI:    A45D26/00 G; A61B18/20; A61N5/067

According to International Patent Classification (IPC) or to both national classification and IPC

**B. FIELDS SEARCHED**

Minimum documentation searched (classification system followed by classification symbols)

    A61B18/20; A61N5/067; A45D26/00

Documentation searched other than minimum documentation to the extent that such documents are included in the fields searched

    Published examined utility model applications of Japan 1922-1996
    Published unexamined utility model applications of Japan 1971-2021
    Registered utility model specifications of Japan 1996-2021
    Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2002-541906 A (KONINKL PHILIPS ELECTRONICS NV) 10 December 2002 (2002-12-10) paragraphs [0005], [0016], [0018], [0020] | 1-3, 7, 9-10 |
| A | | 4-6, 8 |
| A | JP 2007-501047 A (KONINKL PHILIPS ELECTRONICS NV) 25 January 2007 (2007-01-25) entire text, all drawings | 1-10 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **14 December 2021** | **28 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
**Information on patent family members**

International application No.

**PCT/JP2021/042632**

| Patent document cited in search report | | | Publication date (day/month/year) | Patent family member(s) | | | Publication date (day/month/year) |
|---|---|---|---|---|---|---|---|
| JP | 2002-541906 | A | 10 December 2002 | US | 7108690 | B1 | |
| | | | | column 1, line 62 to column 2, line 31, column 5, lines 13-24, column 5, lines 48-60, column 6, line 29 to column 7, line 4 | | | |
| | | | | WO | 2000/062700 | A1 | |
| JP | 2007-501047 | A | 25 January 2007 | US | 2006/0178659 | A1 | |
| | | | | entire text, all drawings | | | |
| | | | | US | 2009/0264872 | A1 | |
| | | | | WO | 2005/011510 | A1 | |
| | | | | CN | 1829481 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005500879 A